# NEW EUROPEAN PATENT SPECIFICATION

(11) **EP 0 828 461 B2**
(45) Date of publication and mention of the opposition decision: **05.07.2006**
(45) Mention of the grant of the patent: 19.02.2003
(21) Application number: 95918298.1
(22) Date of filing: 26.04.1995
(51) Int. Cl.: A61F 2/06, A61M 29/02

(54) **articulated stent with links having kinks**
mit einem geknickten Gelenkmechanismus ausgestatteter Stent
extenseur articulé avec des liaisons coudées

(43) Date of publication of application: 18.03.1998
(60) Divisional application: 01106391.4
(73) Proprietor: MEDINOL LIMITED, Tel Aviv 61581 (IL)
(72) Inventor: PINCHASIK, Gregory, 47414 Ramat Hasharon (IL); RICHTER, Jacob, 47226 Ramat Hasharon (IL)
(74) Representative: Kuhnen & Wacker
(86) International application number: PCT/US1995/005095
(87) International publication number: WO 1996/033671

(56) References cited:
- EP-A- 0 335 341
- EP-A- 0 421 729
- EP-A- 0 541 443
- EP-A- 0 606 165
- EP-A- 0 669 114
- EP-B- 0 759 730
- US-A- 4 856 516
- US-A- 5 102 417

## Description

### FIELD AND BACKGROUND OF THE INVENTION

The present invention relates to stents which are implanted as part of a balloon angioplasty procedure within a bodily conduit of a living animal or a human to maintain patency. In particular, the present invention relates to articulated intravascular stents for delivery through or implantation in a blood vessel having a curved portion.

Intravascular stents having a constricted diameter for delivery through a blood vessel and an expanded diameter for applying a radially outwardly extending force for supporting the blood vessel are known in the art. Articulated intravascular stents for either delivery through a curved blood vessel or implanted therein are also known in the art.

Balloon-expandable stents are commercially available under the trade name Palmaz-Schatz Balloon-Expandable Stents from Johnson & Johnson Intervention Systems Co. The balloon-expandable stent described in EP-A-0 606 165 comprises a hollow tube, open at both stent ends and having a series of slots therein. The ends of the slots are rounded so as to provide smooth: surfaces obviating the abrading of body passageways. However, this stent has the drawback that it shrinks significantly during expansion.

Self-expandable articulated stents are described, for example, in U.S. Patent No. 5,104,404 entitled "Articulated Stent" to Wolff. A prior art self-expandable articulated intravascular stent **10** deployed in a curved blood vessel **16** is now described with reference to Figure 1 which is, in actual fact, Figure 2 of the above referenced U.S. Patent No. 5,104,404. Stent **10** is made up of a number of individual segments **12** articulated by hinges **14** connected at each end to segments **12.** Stent **10** is preferably fabricated from memory shape material, for example, nitinol, and as such is self expandable after delivery from a delivery system described in U.S Patent No. 4,830,003 to Wolff et al. However, these prior art articulated intravascular stents suffer from a number of disadvantages both during delivery through a curved blood vessel and when implanted therein as will now described.

The delivery of stent **10** through curved blood vessel **16** is more complicated than the delivery of a non-articulated stent in that stent **10** has to be angularly oriented such that its hinges **14** are located towards the convex portion of blood vessel **16** so that stent **10** can be flexed inward. In the present example, it will be noted that hinges **14** are located on the same side of segments **12** because blood vessel **16** has only a simple curve in one plane. It can be readily appreciated that delivery of stents through blood vessels which have one or more curved portions which are not in the same plane is even more complicated and generally requires specially constructed stents.

Even when implanted in a curved blood vessel **16,** stents **10** are shown to be lacking In that the gaps between segments **12** render the curved portion of blood vessel **16** without support. Furthermore, the gaps at the convex portion of blood vessel **16** are substantially greater than the gaps at the concave portion thereof, thereby inducing non-uniform and therefore undesirable stresses on blood vessel **16.**

Therefore, it would be highly desirable to have an articulated stent which does not require any particular angular orientation when being delivered through a curved bodily conduit and provides continuous and uniform support for both straight and curved portions of a bodily conduit when implanted.

It would also be highly desirable the structure of a stent does not depend on the particular orientations of curved portions of a blood vessel.
A stent according the first portion of claim 1 is already known from EP 0 335 341 A1.

### SUMMARY OF THE INVENTION

The object of the present Invention is for an articulated stent which can be delivered through a curved bodily conduit using a routine medical procedure and a conventional stent delivery system. Furthermore, the stent provides continuous and uniform support for both straight and curved portions of a bodily conduit when implanted. Still further, the structure of a stent and its support of a bodily conduit do not depend on the orientations of the curved portions of the conduit.

The objective of the present invention is achieved by an articulated stent according to claim 1.

After expansion, the rigid segments of the stent preferably present a fine diamond shaped mesh having 1 mm long sides to provide continuous and uniform support for straight portions of a bodily conduit.

The connectors are implemented as links each having at least one kink. The connectors typically have between 8-24 links to provide continuous and uniform support for both straight and curved portions of a bodily conduit.

The stents have constricted diameters for intraluminal delivery and are then deformed, by the inflation of a balloon forming part of their catheter delivery system, to expanded diameters for applying radially outwardly extending forces for supporting the lumen of bodily conduits. The constricted and expanded diameters of the stents typically fall in the ranges of 1.0-3.5 mm and 3.5-10.0 mm, respectively.

The stents are preferably fabricated from low memory, more plastic than elastic, bio-compatible materials, for example, stainless steel 316L, gold, tantalum, etc. which enables them to be plastically deformed from theirconstricted diameters to their expanded diameters.

A typical stent for implantation in a human coronary artery is 9-21 mm long comprising three to seven 2.2 mm long stent segments connected by two to six 1 mm long connectors such that the ends of the stent subtend between a 45° to 135° angle at a radius of curvature of approximately 9 mm when flexed.

### BRIEF DESCRIPTION OF THE DRAWINGS

The invention is herein described, by way of example only, with reference to the accompanying drawings, wherein:

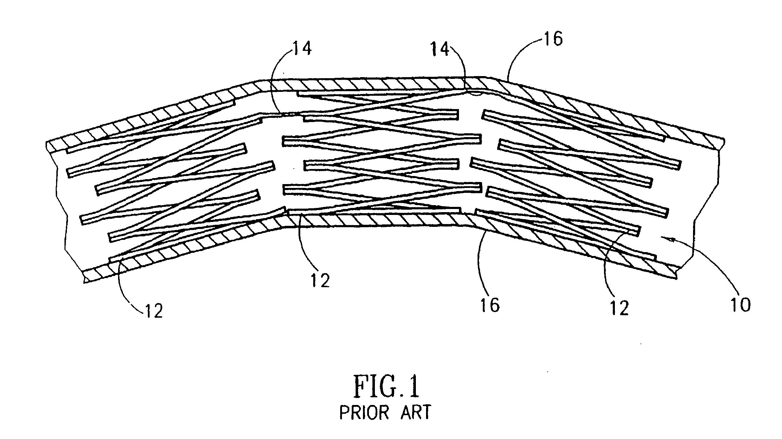

FIG. 1 shows a close-up view of a prior art articulated stent of deployed in a curved blood vessel;
FIGS. 2a and 2b show an articulated stent not covered by the present invention but useful for illustrative purposes in its relaxed and flexed states before plastic deformation;
FIG. 2c shows the expanded stent of Figure 2 after plastic deformation;
FIG. 2d shows the stent of Figure 2 mounted on a catheter in its flexed state;
FIGS. 2e and 2f show the stent of Figure 2 before and after expansion by a balloon forming part of its catheter delivery system;
FIGS. 3a and 3b show a preferred embodiment of an articulated stent, constructed and operative according to the teachings of the present invention, in its relaxed and flexed states before plastic deformation; and
FIG. 3c shows the expanded stent of Figure 3 after plastic deformation.

### DESCRIPTION OF THE PREFERRED EMBODIMENTS

The present invention is of an articulated stent for delivering through a curved bodily conduit, for example, a peripheral or coronary artery of a living animal or a human and implantation therein as part of a balloon angioplasty procedure to maintain patency.

The principles and operation of the articulated stent of the present invention may be better understood with reference to the drawings and the accompanying description.

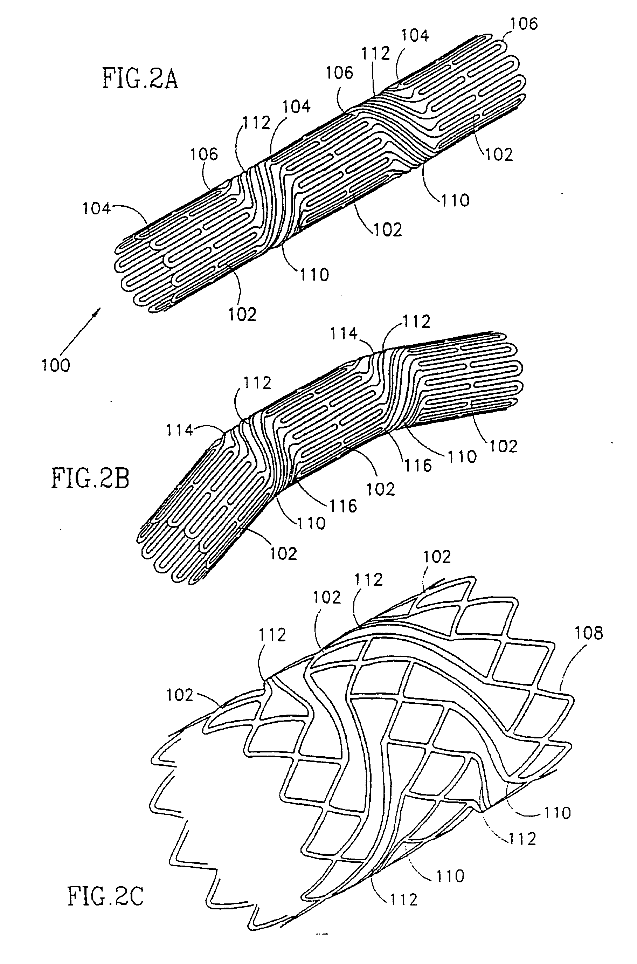

Referring now to the drawings, Figures 2a-2c show an articulated stent not covered by the present invention but useful for illustrative purposes generally designated **100,** generally comprising a number of substantially rigid segments **102** connected by connectors **110.**

Segments **102** are preferably made up to present a fine diamond mesh of interconnected diamond shaped cells **108** having 1 mm sides on expansion as best seen in Figure 2c. Depending on the intended diameter of stent **100,** segments **102** typically comprise between 8-24 diamond shaped cells **108.**

Connectors **110** comprise links **112** connecting a front end **104** to a tail end **106** of adjacent segments **102.** Links **112** preferably extend in a substantially helical fashion between apices of diamond shaped cells **108** at front and rear ends **104** and **106** of adjacent segments **102** such that the number of links **112** equals the number of cells **108.** Links **112** are preferably evenly deployed around perimeters of segments **102** such that connectors **110** can be equally flexed in any direction and to provide continuous and uniform support to both straight and curved portions of a bodily conduit.

Alternate connectors **110** at front and rear ends **104** and **106,** respectively, of a segment **102** preferably have links **112** wound in clockwise and counter clockwise directions. Alternately winding connectors **110** ensures that the rotational displacement of links **112** and adjacent segments **102** relative to the walls of a blood vessel and more importantly the balloon of its delivery system is minimized when stent **100** is expanded.

It is particular feature that connectors **110** have a generally cylindrical configuration when stent **100** is relaxed as best seen in Figure 2a and a differentially stretched and compressed curved configuration when stent **100** is flexed as best seen in Figure 2b. The flexed configuration is brought about by two relatively opposing displacements of links **112.** First, the differential stretching of connectors **110** occurs at the convex portion thereof denoted **114** by links **112** being displaced away from one another. Second, the differential compressing of connectors **110** occurs at the concave portion thereof denoted **116** by links **112** being displaced towards one another.

Stent **100** has a constricted diameter for delivery through a curved bodily conduit as shown in Figures 2a and 2b and an expanded diameter as shown in Figure 2c for supporting a bodily conduit. Stent **100** is preferably fabricated from low memory, more plastic than elastic, bio-compatible material, for example, stainless steel 316L, gold, tantalum, etc. which enables it to be plastically deformed from its constricted diameter to its expanded diameter. The constricted and expanded diameters of stent **100** typically fall in the ranges of 1.0-3.5 mm and 3.5-10.0 mm, respectively.

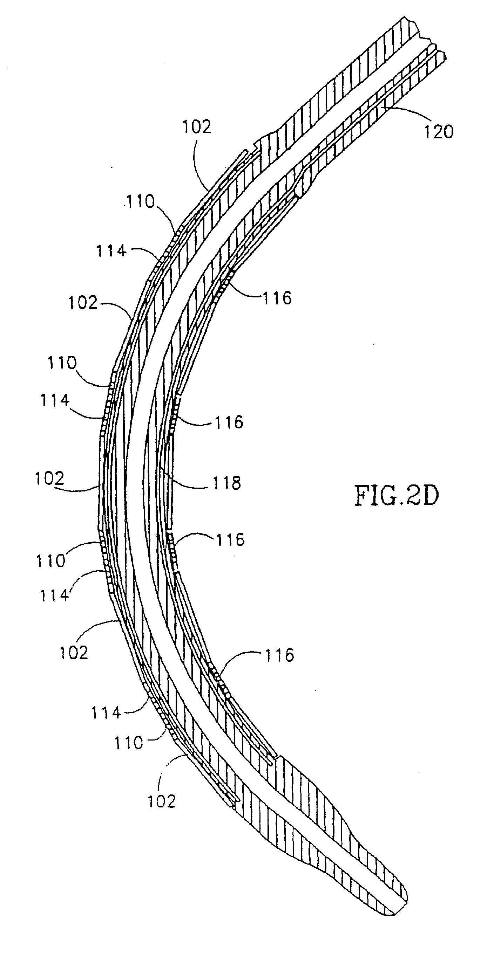

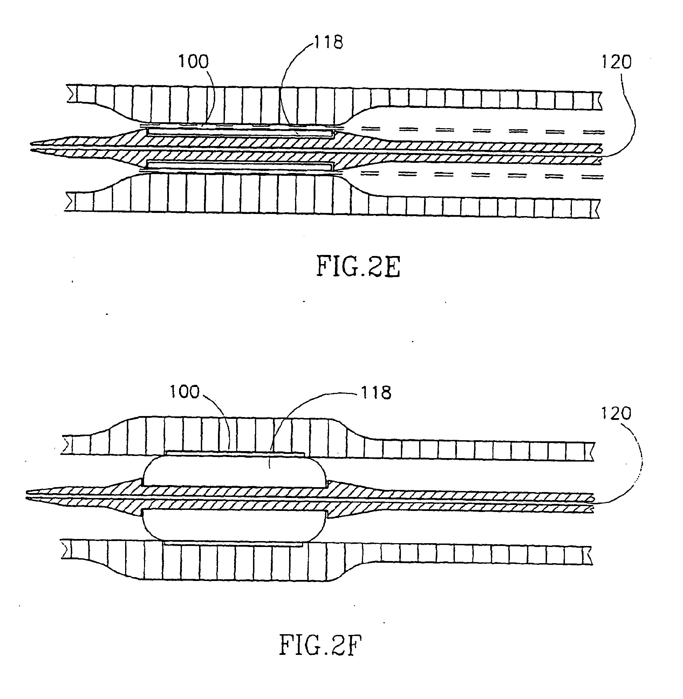

With reference now to Figures 2d-2f, stent **100** is shown overlying a balloon **118** forming part of its catheter delivery system **120.** Stent **100** is mounted on its catheter delivery system **120** in its constricted diameter state shown in Figure 2e for plastic deformation through inflation of balloon **118** to its expanded diameter shown in Figure 2f for supporting the walls of a bodily conduit. An exemplary stent for implantation in a human coronary artery, is typically 15 mm long made up of five 2.2 mm long segments **102** connected by four 1 mm long connectors **110** and capable of flexion such that its ends subtend a 90° angle at a radius of curvature of approximately 9 mm.

The delivery of articulated stent **100** is considerably simpler than the delivery of prior art articulated stent **10** because stent **100** is equally flexible in all direction and therefore does not require a dedicated angular orientation to pass a particular curved portion. This advantage is particularly important for delivery through blood vessels having multiple curved portions. It is a further advantage of stent **100** over prior art stents **10,** that stent **100** provides continuous and uniform support along the entire length of a blood vessel by means of segments **102** and unflexed connectors **110** supporting straight portions thereof while connector portions **114** and **116** supporting convex and concave curved portions thereof, respectively.

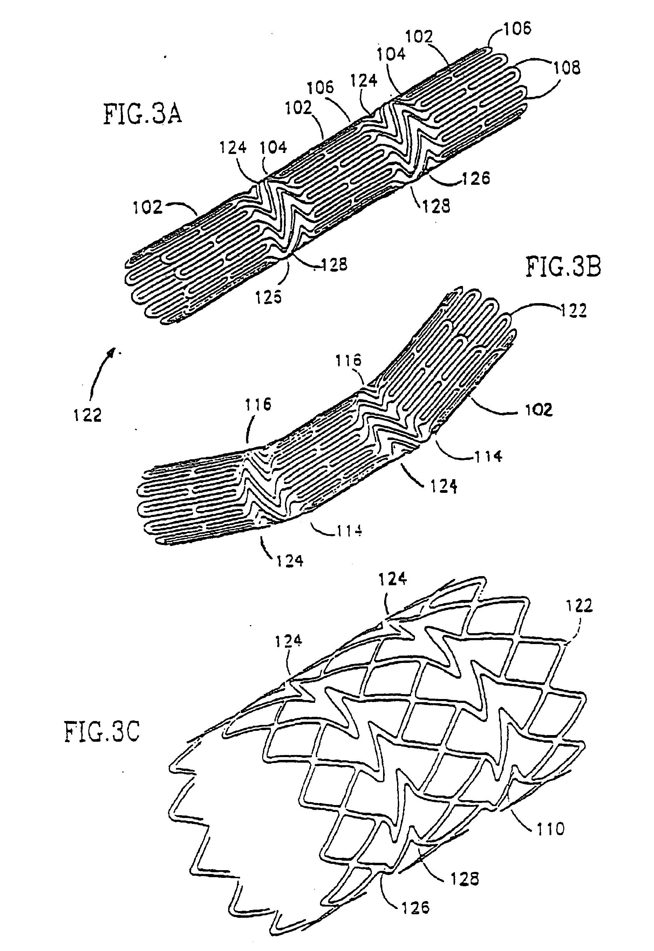

With reference now to Figures 3a and 3b, an articulated stent **122** is shown in which connectors **124** comprise links **126** having one or more kinks **128.** The design of connectors **124** is preferred to that of connector **110** because stent **100** may have a tendency to rupture balloon **118** due to two reasons. First, links **112** overlying the convex portion of balloon **118** have a tendency to be biased inward when stent **100** is flexed. Second, segments **102** display a rotational displacement relative to balloon **118** when stent **100** is expanded.

In this case, the differentially stretched and compressed curved configuration of connector **124** is brought about by two relatively opposing displacements of links **126** as before except that the differential stretching of connectors **124** at convex portion **114** occurs by kinks **128** being somewhat straightened out while the differential compressing of connectors **124** at concave portion **116** occurs by kinks **128** being more acutely bent.

In a similar fashion to stent **100,** stent **122** has a constricted diameter for delivery through a curved bodily conduit as shown in Figures 3a and 3b and an expanded diameter as shown in Figure 3c for supporting a bodily conduit when implanted therein.

While the invention has been described with respect to one embodiment, it will be appreciated that many variations, modifications and other applications of the invention may be made.

## Claims

1. An articulated stent (122), comprising:
- at least two adjacent substantially rigid segments (102), each of said rigid segments (102) comprising a plurality of connected cells (108) each having apices on each end of the segment (102), wherein, upon expansion, each of said rigid segments (102) presents a cylindrical diamond mesh; and
- at least one connector (124) connecting said at least two adjacent segments (102) of the stent (122), wherein each connector comprises a plurality of links (126), each link (126) connecting an apex of a cell (108) disposed on an end of the segment (102) to an apex of an adjacent cell (108) disposed on an end of an adjacent segment (102), with the number of links (126) equalling the number of cells (108),
said links (126) and said at least one connector (124) being flexible,
**characterized in that**
each of said flexible links (126) comprises one or more kinks (128) which, during expansion of the stent, remain inflected.

2. A articulated stent (122) according to claim 1, **characterized in that** said plurality of links (126) include between 8 and 24 links.

3. An articulated stent according to claim 1 or 2, **characterized in that** the stent is made from bio-compatible material capable of a more plastic than elastic deformation.

4. An articulated stent according to claim 1, **characterized in that** said material is gold.

5. An articulated stent according to claim 1, **characterized in that** said material is tantalum.

## Patentansprüche

1. Gelenkstent (122), der folgende Merkmale aufweist:
zumindest zwei benachbarte, im wesentlichen starre Segmente (102), wobei jedes der starren Segmente (102) eine Mehrzahl von verbundenen Zellen (108) aufweist, die jeweils Scheitelpunkte an jedem Ende des Segments (102) aufweisen, wobei, bei Expansion, jedes der starren Segmente (102) ein zylindrisches, rautenförmiges Gitter zeigt; und
zumindest einen Verbinder (124), der die zumindest zwei benachbarten Segmente (102) des Stents (122) verbindet, wobei jeder Verbinder eine Mehrzahl von Verbindungsgliedern (126) aufweist, wobei jedes Verbindungsglied (126) einen Scheitelpunkt einer Zelle (108), die an einem Ende des Segments (102) angeordnet ist, mit einem Scheitelpunkt einer benachbarten Zelle (108) verbindet, die an einem Ende eines benachbarten Segments (102) angeordnet ist, wobei die Anzahl der Verbindungsglieder (126) gleich der Anzahl der Zellen (108) ist,
wobei die Verbindungsglieder (126) und der zumindest eine Verbinder (124) flexibel sind,
**dadurch gekennzeichnet, daß**
jedes der flexiblen Verbindungsglieder (126) eine oder mehrere Knickstellen (128) aufweist, die während einer Expansion des Stents, gebogen bleiben.

2. Gelenkstent (122) nach Anspruch 1, **dadurch gekennzeichnet, daß** die Mehrzahl der Verbindungsglieder (126) zwischen 8 und 24 Verbindungsglieder aufweist.

3. Gelenkstent nach Anspruch 1 oder 2, **dadurch gekennzeichnet, daß** der Stent aus einem biokompatiblen Material gefertigt ist, das zu einer eher plastischen als elastischen Verformung in der Lage ist.

4. Gelenkstent nach Anspruch 1, **dadurch gekennzeichnet, daß** es sich bei dem Material um Gold handelt.

5. Gelenkstent nach Anspruch 1, **dadurch gekennzeichnet, daß** es sich bei dem Material um Tantal handelt.

## Revendications

1. Stent articulé (122), comprenant :
- au moins deux segments adjacents substantiellement rigides (102), chacun desdits segments rigides (102) comprenant une pluralité de cellules reliées (108) possédant chacune des sommets à chaque extrémité du segment (102), où, lors de la dilatation, chacun desdits segments rigides (102) présente une maille en losange cylindrique ; et
- au moins un connecteur (124) reliant lesdits au moins deux segments adjacents (102) du stent (122), où chaque connecteur comporte une pluralité de liaisons (126), chaque liaison (126) reliant un sommet d'une cellule (108) disposé à une extrémité du segment (102) à un sommet d'une cellule adjacente (108) disposé à une extrémité d'un segment adjacent (102), le nombre de liaisons (126) égalant le nombre de cellules (108),
lesdites liaisons (126) et au moins ledit connecteur (124) étant flexibles ;
**caractérisé en ce que**
chacune desdites liaisons flexibles (126) comprend un ou plusieurs coudes (128) qui, lors de la dilatation du stent, restent fléchis.

2. Stent articulé (122) selon la revendication 1, **caractérisé en ce que** ladite pluralité de liaisons (126) comprend entre 8 et 24 liaisons.

3. Stent articulé selon l'une des revendications 1 ou 2, **caractérisé en ce que** le stent est formé de matériau biocompatible capable d'une déformation plus plastique qu'élastique.

4. Stent articulé selon la revendication 1, **caractérisé en ce que** ledit matériau est l' or.

5. Stent articulé selon la revendication 1, **caractérisé en ce que** ledit matériau est le tantale.
